(19) Europäisches Patentamt
European Patent Office
Office européen des brevets

(11) EP 4 684 650 A1

(12) EUROPEAN PATENT APPLICATION

(43) Date of publication:
28.01.2026 Bulletin 2026/05

(21) Application number: 25191122.8

(22) Date of filing: 22.07.2025

(51) International Patent Classification (IPC):
A23L 27/24 (2016.01)      C12N 1/18 (2026.01)
C12R 1/28 (2006.01)      C12R 1/865 (2006.01)

(52) Cooperative Patent Classification (CPC):
A23L 27/24; C12N 1/18; C12R 2001/28;
C12R 2001/865

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 22.07.2024 CN 202410977118

(71) Applicant: Qingdao Chenland Pharmaceutical
Co., Ltd
Qingdao, Shandong 266100 (CN)

(72) Inventors:
• LIANG, Xiaohui
  Qingdao, 266100 (CN)
• YIN, Dongli
  Qingdao, 266100 (CN)
• WANG, Min
  Qingdao, 266100 (CN)
• WANG, Nan
  Qingdao, 266100 (CN)
• LV, Yingfei
  Qingdao, 266100 (CN)
• WANG, Yilei
  Qingdao, 266100 (CN)
• LIU, Tingting
  Qingdao, 266100 (CN)
• MA, Yingxian
  Qingdao, 266100 (CN)
• WANG, Weixia
  Qingdao, 266100 (CN)

(74) Representative: Valet Patent Services Limited
c/o Caya 83713X
Am Börstig 5
96052 Bamberg (DE)

Remarks:
The complete document including Reference
Table(s) and the Sequence Listing(s) can be
downloaded from the EPO website

(54) **TEMPERATURE-SENSITIVE AROMA-PRODUCING SACCHAROMYCES CEREVISIAE AND USE THEREOF IN PREPARATION OF FLAVOR ENHANCERS**

(57) The present application belongs to the technical field of biological engineering, and in particular to a temperature-sensitive aroma-producing *Saccharomyces cerevisiae* and use thereof in preparation of flavor enhancers. The temperature-sensitive aroma-producing *Saccharomyces cerevisiae* is preserved in China Center for Type Culture Collection on May 31, 2024, with the preservation number of CCTCC NO: M20241116. The present application adopts a mixed fermentation method of temperature-sensitive aroma-producing *Saccharomyces cerevisiae* and *Corynebacterium glutamicum* (the preservation number is CCTCC NO: M 2024431) to obtain a fermentation broth containing high concentrations of glutamic acid and nucleotides. Simultaneously, the fermentation broth also contains a variety of functional nutrients, such as other amino acids, organic acids and β-glucan, enabling higher functional nutritional value.

FIG. 1

**Description**

**TECHNICAL FIELD**

The present application belongs to the technical field of bioengineering, and in particular to a temperature-sensitive aroma-producing *Saccharomyces cerevisiae* and use thereof in preparation of flavor enhancers.

**BACKGROUND ART**

With the improvement of living standards, people's demand for natural flavor enhancers is increasing. At present, a variety of production methods for natural flavor enhancers have emerged, but there are still some shortcomings: (1) *Corynebacterium glutamicum* needs pure strain fermentation, the product is monosodium glutamate, with a single component and insufficient flavor; and (2) two-step fermentation technology has been adopted by some people, mainly using mold and bacterial fermentation, with complex steps, easy bacterial contamination and high cost.

China invention patent application 202180054828.3 provides a taste enhancing composition comprising a plant from the Umbelliferae family and having a taste-effective glutamate content, and a method of producing a taste enhancer by reducing biotin in fermentable carbohydrates prior to bacterial fermentation. The method produces the taste enhancer from a carrot juice, carrot puree and carrot pomace or a combination thereof used as a raw material through microbial fermentation using *Corynebacterium glutamicum, Corynebacterium ammoniagenes, Corynebacterium casei, Brevibacterium lactofermentum, Bacillus subtilis* or combinations thereof, etc. In addition to the glutamate, the taste enhancer also contains organic acids including lactic acid, citric acid, acetic acid, succinic acid, and combinations thereof. Although the method improves the fermentation efficiency, the problems of simple components and insufficient mouthfeel of the flavor enhancer are still not solved. In addition, the taste enhancer still needs to be added with exogenous auxiliary components such as sodium chloride (salt), ribonucleotide, inosine monophosphate, guanosine monophosphate, monosodium glutamate, yeast, amino acid blend, peptide, arginine hydrochloride, arginine ammonium chloride, lysine hydrochloride, lysine-ornithine hydrochloride and the like. The addition of these components destroys the pure natural and green image of the product. Invention patent WO 2015/020292 ferments plant proteins (such as soy protein, wheat protein, com protein, etc.) through mold (such as *Aspergillus sojae* CJCC_080124P (KCCM11026P)) to produce delicious amino acids, polypeptides, etc., and performs further fermentation by using bacteria (such as *Corynebacterium ammoniatum* CJIP009 (KCCM-10226), *Corynebacterium glutamicum, Bacillus,* etc.) after adding carbon sources, phosphorus sources, and trace elements, to produce more umami substances. Although this method can increase the variety of fermentation products and improve umami level and flavor, it has problems such as cumbersome steps and high production cost. Chinese invention patent 201410790020.3 discloses a *Saccharomyces cerevisiae* strain with excellent autolysis resistance. The cell wall of the strain is twice as thick as that of the original strain, the data from simulated autolysis experiment data and the results of scanning electron microscope after 60 hours both show that the recombinant strain has significantly better autolysis performance that the original strain, and has good application value in improving beer flavor, foam stability and other qualities. Since this strain is genetically modified, it cannot be used in food production at present. Chinese invention patent application 201610538817.3 discloses an autolysis process method for producing a highly-active substance, yeast autolysate, which mixing a specific single-cell protein that is not sterilized and maintains the original endogenous enzyme activity with a yeast in proportion, autolyzing and then drying and sterilizing, where the autolyzing includes a low-temperature stage and a high-temperature stage, and is kept at 45 to 60°C for 20-60 minutes in the low-temperature stage, and kept at 75 to 95°C for 30-120 minutes in the high-temperature stage. This technology has the problems of high processing temperature and high energy consumption, and is easy to destroy the flavor of the product.

Therefore, the key issue that needs to be solved urgently is to establish a preparation technology for natural flavor enhancers with low cost, simple operation steps and rich flavor.

**SUMMARY**

In view of the problems existing in the prior art, the present application provides a temperature-sensitive aroma-producing *Saccharomyces cerevisiae* and use thereof in preparation of flavor enhancers. The *Saccharomyces cerevisiae* may be used to prepare flavor enhancers. The glutamic acid, flavor nucleotides and other products may be obtained from a carrot juice as a main raw material through mixed fermentation of the *Saccharomyces cerevisiae* and *Corynebacterium glutamicum*, to obtain the natural flavor enhancer with higher umami level and richer flavor while reducing production costs and improving production efficiency.

The technical solution adopted by the present application for solving the technical problem is temperature-sensitive aroma-producing *Saccharomyces cerevisiae,* preserved in China Center for Type Culture Collection on May 31, 2024, with the preservation number of CCTCC NO: M 20241116.

The present application further provides a use of the *Saccharomyces cerevisiae* in preparation of a flavor enhancer, where the flavor enhance is prepared from a carrot juice as the main raw material through mixed fermentation of the *Saccharomyces cerevisiae* and *Corynebacterium glutamicum.*

The present application further provides a preparation method of the flavor enhancer, including:

(1) inoculating activated temperature-sensitive aroma-producing *Saccharomyces cerevisiae* into a sterilized fermentation medium, where a fermentation temperature is controlled at 28±1°C, a pH range is 4.5-6.5, dissolved oxygen is maintained at 10%-30% through combined control of rotation speed and ventilation, and a fermentation time is 12 h-24 h;

(2) supplementing into a fermentation tank a carrot juice and a concentrated sugar solution which are sterilized, to make total sugar reach 7% (w/v) or above, when a wet weight of the *Saccharomyces cerevisiae* reaches 50 g/L or above and the total sugar drops to 0.5% (w/v) or below, heating to 40°C-75°C, maintaining for 30 min-120 min, then rapidly cooling to 30°C, and inoculating freshly activated *Corynebacterium glutamicum* CCTCC NO: M 2024431; and performing mixed fermentation for 12h-24 h, where a temperature for the mixed fermentation is controlled at 30°C-33°C, the dissolved oxygen is maintained at 30%-50% through combined control of rotation speed and ventilation, and pH is controlled at 6.5-8.0 during the fermentation; and

(3) heating to 100°C after the fermentation is terminated, and maintaining for 10 min-60 min.

Preferably, during the mixed fermentation, when the total sugar drops to 0.2%-2.0% (w/v), fed-batch sugar supplementation is begun, and fed-batch sugar is one or a combination of two or more, in any ratio, selected from glucose, sucrose, fructose, com syrup and fructose syrup, and in a sugar solution for the fed-batch sugar supplementation, in parts by weight, sugar : water = 1:1.

Preferably, the fermentation medium uses a carrot juice as the main raw material, and includes the following components in mass ratio: 500-1,000 g/kg of carrot juice, 5.0-15 g/kg of dipotassium hydrogen phosphate, 0.05-2.0 g/kg of magnesium sulfate heptahydrate, 0.005-0.2 g/kg of ferrous sulfate heptahydrate, 0.001-0.2 g/kg of manganese sulfate monohydrate, 0-3.0 g/kg of aspartic acid, and 0-3.0 g/kg of threonine.

Preferably, in the fermentation medium, an initial total sugar content is controlled within a range of 3%-5% (w/v), and an initial pH is 4.5-6.5, and the fermentation medium is sterilized after uniform mixing, under a sterilization condition of 115°C-121°C for a time of 15 min-30 min.

Preferably, the solids content of the carrot juice is 6.0°Brix.

The present application further provides a flavor enhancer prepared by using the method, and the flavor enhancer includes a product of mixed fermentation of the temperature-sensitive aroma-producing *Saccharomyces cerevisiae* and *Corynebacterium glutamicum* CCTCC NO: M 2024431.

Preferably, in the product, a glutamic acid concentration is 80-156 g/L, and a total nucleotide concentration is 250 mg/L or above.

Preferably, the product further includes β-glucan and organic acids.

Compared with the prior art, the beneficial effects of the present application are reflected in that: the temperature-sensitive aroma-producing *Saccharomyces cerevisiae* provided by the present application has the characteristic of high-temperature autolysis when growing in the carrot juice, that is, it may grow and proliferate normally at 25°C-28°C, where the growth is best at 28°C, the growth is significantly inhibited when the temperature exceeds 30°C, and when the temperature reaches 35°C or above, the fungal cells begin to undergo wall disruption and autolysis, and simultaneously release intracellular contents such as RNAs and proteins.

The present application adopts a method of performing mixed fermentation using the temperature-sensitive aroma-producing *Saccharomyces cerevisiae* and *Corynebacterium glutamicum* (preservation number is CCTCC NO: M 2024431; preserved in China Center for Type Culture Collection on March 8, 2024; the address of the preservation unit is: Wuhan University, Wuhan City, Hubei Province).

) to obtain a fermentation broth containing high concentrations of glutamic acid and nucleotides. Simultaneously, the fermentation broth also contains a variety of functional nutrients such as other amino acids, organic acids, β-glucan, etc. In the fermentation broth obtained at the endpoint of the fungus-bacterium fermentation (mixed fermentation), the glutamic acid concentration can reach 80 g/L or above (up to 156 g/L), and the total nucleotide concentration can reach 250 mg/L or above. The two types of substances synergistically increase the umami level, enabling stronger umami than a pure glutamic acid fermentation broth, rich and harmonious taste, richer material composition, and higher functional nutritional value.

**BRIEF DESCRIPTION OF DRAWINGS**

FIG. 1 shows a colony morphology of temperature-sensitive aroma-producing *Saccharomyces cerevisiae* provided in embodiments of the present application; and

FIG. 2 is a growth curve of the temperature-sensitive aroma-producing *Saccharomyces cerevisiae* provided in embodiments of the present application.

## DETAILED DESCRIPTION OF EMBODIMENTS

In order to facilitate the understanding of the present application, more detailed description of the present application will be made in conjunction with the drawings and specific embodiments. However, the present application may be realized in many different forms and is not limited to the embodiments described in this specification. On the contrary, an objective of providing these embodiments is to make a more thorough and comprehensive understanding of the content disclosed in the present application.

Example 1: The present application provided a temperature-sensitive aroma-producing *Saccharomyces cerevisiae,* and the *Saccharomyces cerevisiae* was derived from the sea.

I. Screening and Isolation of Strain

(1) Isolation and purification of strain: in an early stage, the sea area of Qingdao, Shandong Province was selected for the collection of representative seaweeds (mainly including *Saccharina japonica* (seatangle), *Sargassum* (gulfweed), and *Enteromorpha prolifera,* and the like), and the collected samples were then quickly placed in sterile sampling bottles. The seaweed samples were subjected to multi-point sampling under a sterile condition in the laboratory, then placed in sterile normal saline for shaking and soaking, and gradiently diluted, after a certain period of time, to prepare bacterial solution samples of different concentrations. The bacterial solution samples of individual gradient concentrations were sequentially coated on a nutrient agar (NA) medium, an MRS (de Man, Rogosa and Sharpe) agar medium, a potato dextrose agar (PDA) medium and a yeast extract peptone dextrose agar (YPDA) medium, cultured under different conditions, and subjected to isolation of microbial resources such as bacteria, yeast and mold. Among them, 10 single colonies, including the strain, were isolated from the YPDA plate, included 3 yeast strains, which were purified by means of plate streaking, and streaked on the slanted surface of a test tube for preservation at 4°C.

(2) Screening of strains: the three yeast strains, including Y01, were initially selected, and subjected to tests and analysis of the growth and reproductive capacity in a specific medium (sterilized carrot juice), sensory characteristics of fermentation broth, RNA contents of the strains and mixed fermentation performance. The final results showed that compared with other strains, this strain was temperature-sensitive, and could rapidly reproduce in the carrot juice medium, and the fermentation broth had an obvious and pleasant aroma. Meanwhile, the strain had a high RNA content and could coexist with *Corynebacterium glutamicum* (the preservation number is CCTCC NO: M 2024431) and be used for synergetic fermentation, so this strain was selected for the preparation of the flavor enhancer.

II. Molecular Biological Identification and Biological Preservation

The strain obtained by the screening was identified by using a molecular biology technique, and the 18S rDNA nucleotide sequence was as shown in SEQ ID No. 1 in the Sequence Listing. Through a sequence homology analysis of the National Center for Biotechnology Information (NCBI) of the United States, the 18S rDNA nucleotides of the aroma-producing *Saccharomyces cerevisiae* in the present application were compared using a BLAST tool. The comparison results indicated that Y01 belonged to *Saccharomyces cerevisiae.*

The *Saccharomyces cerevisiae* strain Y01 was preserved in the China Center for Type Culture Collection on May 31, 2024, with the preservation number CCTCC NO: M 20241116. The address of the preservation unit is: Wuhan University, Wuhan City, Hubei Province.

III. Determination of Culture Characteristics, Biological Characteristics and Functional Characteristics

1. Biological Characteristics:

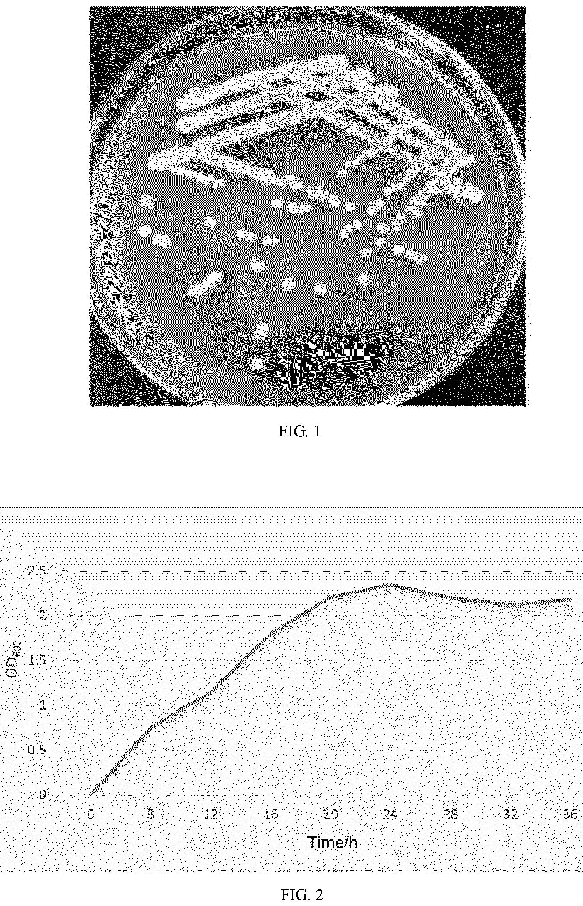

The strain is easy to culture, grows and reproduces rapidly, and proliferates in a budding reproduction manner under a normal culture condition. When cultured on the YPDA medium, typical colonies may be formed after about 30 h. The colonies are opalescent, flat, and moist and easy to pick, as shown in FIG. 1. An obvious fruity and floral aroma may be smelled after a long-term culture. The strain is adapted to a wide range of pH, and may grow at the condition of pH 3.5-7.5. Namely, the strain may maintain a high growth and reproduction rate under the optimal growth pH (~7.0) of *Coryne-*

*bacterium glutamicum.*

2. Culture Characteristics:

The experimental study found that the yeast could use a variety of carbon sources, including glucose, fructose, sucrose, mannitol, galactose, and the like. Among them, the utilization efficiency of glucose is the highest, and the utilization speeds of the fructose and sucrose were also faster. The strain grew normally at 25°C-30°C, and proliferated best at around 28°C. When the temperature exceeded 30°C, the reproduction rate began to decrease, and the fungal cells began to deform. When the temperature continued to rise to 35°C or above, the cells began to autolyze, so the strain was temperature sensitive. The strain grew under the condition of pH 3.5-7.5, and grew best at pH 4.5-5.0. When cultured in a shake flask, the maximum fungal concentration exhibits a trend of first increasing and then decreasing with the increase in rotation speed, and the optimum rotation speed was 180 r/min. When cultured in pure carrot juice (6.0°Brix), the lag phase of the strain was about 4 h, and the logarithmic phase began from 6 h to 8 h, in which the strain rapidly proliferated. The biomass of the strain reached the maximum at 14 h-16 h, and the growth rate slowed down, gradually entering a stable phase.

FIG. 2 shows a growth curve of the strain under the optimal temperature and rotation speed conditions (the medium formula is in parts by weight: 1% yeast extract, 2% peptone, and 2% glucose).

3. Determination of Functional Characteristics:

The yeast strain of the present application has good aroma production characteristics. When cultured alone in a carrot juice, the culture solution had an obvious fruity and floral aroma. When mixed with *Corynebacterium glutamicum* for fermentation, in addition to the fruity and floral aroma, new aromas such as milky and sweet fragrances were further provided.

Determination of RNA Content:

(1) the yeast was cultured, centrifuged and dried to obtain dry yeast powder;
(2) about 2 g of the dry yeast powder ($m_0$) was weighed, suspended in 12 mL of 0.04 mol/L sodium hydroxide solution, and uniformly ground in a mortar;
(3) the fungal suspension was transferred to a test tube, heated in a boiling water bath for 30 min, cooled, transferred to a centrifuge tube, and centrifuged at 4,000 r/min for 10 min;
(4) the supernatant was slowly injected into 4 mL of acidic ethanol, and mixed uniformly while adding, stood after the adding, until the RNA was completely precipitated, and centrifuged at 3,000 r/min for 5 min, and the supernatant was discarded;
(5) the precipitate was washed twice with 95% ethanol, and then the precipitate was washed once with diethyl ether;
(6) the precipitate was transferred using diethyl ether to a Buchner funnel for suction filtration, the precipitate was dried in air, and the weight ($m_1$, g) of the crude RNA was weighed; and
(7) the sample was prepared into a solution of 5-50 $\mu$g/L, which was then measured to obtain the absorbance values at 260 nm and 280 nm, and the RNA content (%) was calculated, where the calculation formula was as follows:

$$RNA\ content\ (\%) = (RNA\ content \times m_1)/(1000 \times m_0) \times 100\%;$$

$$RNA\ content\ (\mu g/g) = (OD_{260} \times dilution\ multiple)/(0.024 \times L);$$

In the formula: $OD_{260}$ is the absorbance of the sample, $M_0$ is the mass of dry yeast powder, g; L is the thickness of a colorimetric cup, and 0.024 is the absorbance of 1 $\mu$g RNA/mL.
After the measurement, the RNA content of the yeast strain of the present application can reach 10% (w/w) or above, while the RNA contents of synchronously cultured control strains (commercial bread yeast strains BY1 and BY2) were only 5% to 7% (w/w).
Example 2: The present example provided a method for preparing a natural flavor enhancer by mixed fermentation using a carrot juice with aroma-producing *Saccharomyces cerevisiae* and *Corynebacterium glutamicum*, including the following specific steps:
(1) Raw Material Preparation and Strain Activation

(i) preparation of carrot juice

selecting fresh, non-soft and rot-free carrots as raw materials, cleaning, peeling, trimming and cutting into pieces, pre-cooking for 5-10 min, transferring to a juicer for pulping, performing juice-residue separation on the obtained carrot pulp, sterilizing, and storing for use after being tested to be qualified, see Table 1;

**Table 1: Carrot Juice Quality Test Items and Results**

| Number | Test Item | Test Result |
|---|---|---|
| 1 | Sensory quality | Fresh carrot color, typical carrot flavor, no visible impurities to the naked eye |
| 2 | Soluble solids (20°C refractometry, °Brix) | 7.5 |
| 3 | Total sugar, % (w/w) | 5.42 |
| 4 | Total acid (based on Citric Acid), % (w/w) | 0.1 |
| 5 | pH value | 5.10 |
| 6 | Amino nitrogen, mg/100 g | 15.7 |

(ii) Strain Activation

preparing a yeast seed medium: 300 g/L carrot juice (solids of 6.0°Brix), with an initial total sugar of 1.65% (w/w), which was sterilized at 115°C for 15 min; after sterilizing, cooling to an appropriate temperature, inoculating aroma-producing *Saccharomyces cerevisiae* according to an inoculation amount of 1% (v/v), culturing at 28°C and 180 r/min for 24 h, subculturing the obtained strain once in the above medium, with an inoculation amount of 5% (v/v), for a culture time of 18 h for later use; and

preparing a *Corynebacterium glutamicum* seed medium: 200 g/L of carrot juice (solids of 6.0°Brix), 20 g/L of sugar (calculated as monosaccharide-glucose), 0.3 g/L of threonine, 3.0 g/L of dipotassium hydrogen phosphate, 0.5 g/L of magnesium sulfate heptahydrate, 0.01 g/L of ferrous sulfate heptahydrate, and 0.01 g/L of manganese sulfate monohydrate, where an initial pH was adjusted to 7.5 by using ammonia water, and an initial total sugar was 2.9% (w/v); mixing uniformly and then sterilizing under a sterilization condition of 115°C for 30 min; after sterilizing, cooling to an appropriate temperature, inoculating *Corynebacterium glutamicum* (CCTCC No. M 2024431) according to an inoculation amount of 1.2%, culturing at 32°C and 200 r/min for 16 h; subculturing the obtained strain once in the above medium, with an inoculation amount of 5% (v/v), for a culture time of 12 h for later use;

(2) Fermentation

(i) preparing a fermentation medium: 500 g/kg of carrot juice (solids of 6.0°Brix), 5.0 g/kg of dipotassium hydrogen phosphate, 0.5 g/kg of magnesium sulfate heptahydrate, 0.1 g/kg of ferrous sulfate heptahydrate, 0.01 g/kg of manganese sulfate monohydrate, 1.5 g/kg of aspartic acid, and 1.5 g/kg of threonine, which was sterilized at 121°C for 20 min, and

separately preparing a concentrated sugar solution (in parts by weight, sugar: water = 1:1), separately sterilizing, and storing in a storage tank for later use; and

(ii) inoculation fermentation

inoculating the yeast seed solution obtained from (i) in the above step (1) into the fermentation medium according to the inoculation amount of 3% (v/v), where during the fermentation process, pH was controlled at 4.5, the dissolved oxygen was maintained at 10% through combined control of the rotation speed and ventilation, and a fermentation time was 12 h; supplementing the carrot juice ($\geq$ 30°Brix) and the concentrated sugar solution which were sterilized in advance, into a fermentation tank through a pipeline to make the total sugar reach 7% (w/v), heating to 40°C, maintaining for 120 min, then rapidly cooling to 30°C, and inoculating freshly activated *Corynebacterium glutamicum,* where during the fermentation process, the temperature was controlled at 31±1°C, the dissolved oxygen was maintained at 30% through combined control of the rotation speed and ventilation, and the pH was controlled at 6.5 during the fermentation process, and continuing the fermentation for 24 h, during which fed-batch sugar supplementation started when the total sugar dropped to 0.2% (w/v), then heating to 100°C after the fermentation was terminated, and maintaining for 60 min; and

(3) Detection of Fermentation Broth

testing the fermentation broth obtained in step (2) in Example 2 for relevant indicators after removing the strain. The test results are as shown in Table 2.

**Table 2: Detection of Components of Fermentation Broth Obtained in Example 2**

| Component | Test Result-Fungus-Bacterium Fermentation |
|---|---|
| Glutamic acid (g/L) | 85 |
| 5'-Inosine monophosphate (5'-IMP, mg/L) | 25 |
| 5'-Guanosine monophosphate (5'-GMP, mg/L) | 50 |
| 5'-Adenosine monophosphate (5'-AMP, mg/L) | 110 |
| 5'-Xanthosine monophosphate (5'-XMP, mg/L) | 78 |
| Nucleotide (mg/L, 5'-IMP+5'-GMP+5'-AMP+5'-XMP) | 263 |
| Lactic acid (g/L) | 12.7 |
| Yeast $\beta$ -glucan (mg/L) | 55 |

Example 3: The present example provided a method for preparing a natural flavor enhancer by mixed fermentation using a carrot juice with aroma-producing *Saccharomyces cerevisiae* and *Corynebacterium glutamicum,* including the following specific steps:

(1) Raw Material Preparation and Strain Activation, same as in Example 1;

(2) Fermentation

(i) preparing a fermentation medium: 700 g/kg of carrot juice, 3.5 g/kg of dipotassium hydrogen phosphate, 0.5 g/kg of magnesium sulfate heptahydrate, 0.1 g/kg of ferrous sulfate heptahydrate, 0.01 g/kg of manganese sulfate monohydrate, 0.5 g/kg of aspartic acid, and 0.5 g/kg of threonine, which was sterilized at 121°C for 20 min, and separately preparing a concentrated sugar solution (in parts by weight, sugar: water = 1:1), separately sterilizing, and storing in a storage tank for later use; and

(ii) inoculation fermentation

inoculating the yeast seed solution obtained from (i) in step (1) above into the fermentation medium according to the inoculation amount of 5% (v/v), where during the fermentation process, pH was controlled at 5.5, the dissolved oxygen was maintained at 25% through combined control of the rotation speed and ventilation, and a fermentation time was 12 h, supplementing the carrot juice ($\geq$ 30°Brix) and the concentrated sugar solution which were sterilized in advance, into a fermentation tank through a pipeline to make the total sugar reach 12% (w/v), heating to 75°C, maintaining for 30 min, then rapidly cooling to 30°C, and inoculating freshly activated *Corynebacterium glutamicum,* where during the fermentation process, the temperature was controlled at 31±1°C, the dissolved oxygen was maintained at 50% through combined control of the rotation speed and ventilation, and the pH was controlled at 7.5 during the fermentation process, and continuing the fermentation for 24 h, during which fed-batch sugar supplementation started when the total sugar dropped to 0.2% (w/v), then heating to 100°C after the fermentation was terminated, and maintaining for 10 min; and

(3) Detection of Fermentation Broth

testing the fermentation broth obtained in step (2) in Example 3 for relevant indicators after removing the strain. The test results are as shown in Table 3.

**Table 3: Detection of Components of Fermentation Broth Obtained in Example 3**

| Component | Test Result- Fungus-Bacterium Fermentation |
|---|---|
| Glutamic acid (g/L) | 102 |
| 5'-IMP (mg/L) | 45 |
| 5'-GMP (mg/L) | 50 |
| 5'-AMP (mg/L) | 156 |
| 5'-XMP (mg/L) | 75 |
| Nucleotide (mg/L, 5'-IMP+5'-GMP+5'-AMP+5'-XMP) | 326 |
| Lactic acid (g/L) | 2.5 |

(continued)

| Component | Test Result- Fungus-Bacterium Fermentation |
|---|---|
| Yeast β -glucan (mg/L) | 97 |

Example 4: The present example provided a method for preparing a natural flavor enhancer by mixed fermentation of a carrot juice with aroma-producing *Saccharomyces cerevisiae* and *Corynebacterium glutamicum,* including the following specific steps:

(1) Raw Material Preparation and Strain Activation, same as in Example 1;

(2) Fermentation

(i) preparing a fermentation medium (800 g/kg of carrot juice, 3.5 g/kg of dipotassium hydrogen phosphate, 0.5 g/kg of magnesium sulfate heptahydrate, 0.1 g/kg of ferrous sulfate heptahydrate, 0.01 g/kg of manganese sulfate monohydrate, 0.5 g/kg of aspartic acid, and 0.5 g/kg of threonine), which was sterilized 121°C, for 20 min; and separately preparing a concentrated sugar solution (in parts by weight, sugar : water = 1:1), separately sterilizing, and storing in a storage tank for later use; and

(ii) inoculation fermentation: inoculating the yeast seed solution obtained from (i) in step (1) above into the fermentation medium according to the inoculation amount of 5% (v/v), where during the fermentation process, pH was controlled at 5.5, the dissolved oxygen was maintained at 30% through combined control of the rotation speed and ventilation, and a fermentation time was 16 h, supplementing the carrot juice (≥ 30°Brix) and the concentrated sugar solution which were sterilized in advance, into a fermentation tank through a pipeline to make the total sugar reach 10% (w/v), heating to 55°C, maintaining for 30 min, then rapidly cooling to 30°C, and inoculating freshly activated *Corynebacterium glutamicum,* where during the fermentation process, the temperature was controlled at 31 ±1°C, the dissolved oxygen was maintained at 30% through combined control of the rotation speed and ventilation, and the pH was controlled at 7.0 during the fermentation process, and continuing the fermentation for 24 h, during which fed-batch sugar supplementation started when the total sugar dropped to 1%, then heating to 100°C after the fermentation was terminated, and maintaining for 20 min; and

(3) Detection of Fermentation Broth
testing the fermentation broth obtained in step (2) in Example 4 for relevant indicators after removing the strain. The test results are as shown in Table 4.

**Table 4: Detection of Components of Fermentation Broth Obtained in Example 4**

| Component | Test Result- Fungus-Bacterium Fermentation |
|---|---|
| Glutamic acid (g/L) | 108 |
| 5'-IMP (mg/L) | 42 |
| 5'-GMP (mg/L) | 56 |
| 5'-AMP (mg/L) | 218 |
| 5'-XMP (mg/L) | 78 |
| Nucleotide (mg/L, 5'-IMP+5'-GMP+5'-AMP+5'-XMP) | 394 |
| Lactic acid (g/L) | 3.2 |
| Yeast β -glucan (mg/L) | 118 |

Example 5: The present example provided a method for preparing a natural flavor enhancer by pure fermentation of a carrot juice with *Corynebacterium glutamicum* Gu2#, including the following specific steps:

(1) Raw Material Preparation and Strain Activation

(i) preparing the carrot juice: selecting fresh, non-soft and rot-free carrots as raw materials, cleaning, peeling, trimming and cutting into pieces, pre-cooking for 5-10 min, transferring to a juicer for pulping, performing juice-residue separation on the obtained carrot pulp, sterilizing, and storing for use after being tested to be qualified; and

(ii) Strain Activation

activating primary strain: preparing a primary seed medium (g/L) according to the following formula under the following culture conditions:

300 g/L of carrot juice, 20 g/L of sugar (calculated as monosaccharide-glucose), 0.3 g/L of threonine, 3.0 g/L of dipotassium hydrogen phosphate, 0.5 g/L of magnesium sulfate heptahydrate, 0.01 g/L of ferrous sulfate heptahydrate, 0.01 g/L of manganese sulfate monohydrate, where an initial pH was adjusted to 7.5 by using ammonia water, and an initial total sugar was 3.35% (w/v); mixing uniformly and then sterilizing, under a sterilization condition of 115°C for 15 min, cooling to an appropriate temperature after sterilization, for inoculation: the inoculation amount: 2% (v/v), liquid amount: 15% (v/v), and culture conditions: 32°C, 200 r/min, 14 h; and

activating secondary strains: preparing a secondary seed medium (g/L) according to the following formula under the following culture conditions:

400 g/L of carrot juice, 30 g/L of sugar (calculated as monosaccharide-glucose), 0.5 g/L of threonine, 5.0 g/L of dipotassium hydrogen phosphate, 0.5 g/L of magnesium sulfate heptahydrate, 0.01 g/L of ferrous sulfate heptahydrate, 0.01 g/L of manganese sulfate monohydrate, where an initial pH was adjusted to 7.5 by using ammonia water, and an initial total sugar was 5.06% (w/v); mixing uniformly and then sterilizing under a sterilization condition of 115°C for 15 min, cooling an appropriate temperature after sterilization, for inoculation: the inoculation amount: 10% (v/v), liquid amount: 15% (v/v), and culture condition: 32°C, 200 r/min, 10 h;

(2) Fermentation

preparing an initial fermentation medium: 800 g/kg of carrot juice, 55 g/kg of sugar (calculated as monosaccharide-glucose), 0.5 g/kg of threonine, 3.5 g/kg of dipotassium hydrogen phosphate, 0.5 g/kg of magnesium sulfate heptahydrate, 0.01 g/kg of ferrous sulfate heptahydrate, 0.001 g/kg of manganese sulfate monohydrate, and an anti-foaming agent added according to the need, where the initial total sugar content in the medium was 9.8 % (w/v), mixing uniformly and then sterilizing under a sterilization condition of 115°C for 30 min;, cooling to an appropriate temperature after sterilization, adjusting the initial pH to 7.0 with ammonia water, and inoculating with an inoculation amount of 10% for fermentation, where the fermentation temperature was controlled at 30°C, the dissolved oxygen was maintained at 50% through combined control of the rotation speed and ventilation, a fermentation time was 36 h, and a key for pH control during the fermentation process was: pH being controlled at 6.5-7.0 in the logarithmic phase, pH being controlled at 7.0-7.2 in acid production phase, and pH being controlled at around 7.5 at 6-8 h before the termination; and during the fermentation, fed-batch sugar supplementation started when the total sugar content dropped to 1.5% (w/v), to maintain the total sugar content to be not less than 1% (w/v), and a sugar-acid conversion rate was controlled at about 50% during the process; and

(3) Detection of Fermentation Broth

testing the fermentation broth obtained in step (2) in Example 5 for relevant indicators after removing the strain. The test results are as shown in Table 5.

**Table 5: Detection of Components of Fermentation Broth Obtained in Example 5**

| Component | Test Result-Fungus-Bacterium Fermentation |
|---|---|
| Glutamic acid (g/L) | 125 |
| 5'-IMP (mg/L) | - |
| 5'-GMP (mg/L) | 25 |
| 5'-AMP (mg/L) | 150 |
| 5'-XMP (mg/L) | - |
| Nucleotide (mg/L, 5'-IMP+5'-GMP+5'-AMP+5'-XMP) | 175 |
| Lactic acid (g/L) | 3.1 |
| Yeast $\beta$ -glucan (mg/L) | - |

The compositions and contents of the final fermentation broths obtained by the two production processes of glutamic acid pure fermentation in Example 5 and fungus-bacterium mixed fermentation were compared. The results are shown in Table 6.

**Table 6: Comparison of Components in Single-Bacterium Fermentation Broth (*Corynebacterium glutamicum*) and Fungus-Bacterium Fermentation Broth**

| Component | Single-Bacterium Fermentation-Example 5 (*Corynebacterium glutamicum* Gu2#) | Fungus-Bacterium Fermentation-Example 4 (*Corynebacterium glutamicum* Gu2# + *Saccharomyces cerevisiae* Y01) |
|---|---|---|
| Glutamic acid (g/L) | 125 | 108 |
| Aspartic acid (g/L) | 0.15 | 0.35 |
| 5'-IMP (mg/L) | 0 | 42 |
| 5'-GMP (mg/L) | 25 | 56 |
| 5'-AMP (mg/L) | 150 | 218 |
| 5'-XMP (mg/L) | - | 78 |
| Nucleotide (mg/L, 5'-IMP+5'-GMP+5'-AMP+5'-XMP) | 175 | 394 |
| Alanine (g/L) | 0.38 | 0.12 |
| Proline (g/L) | 0.38 | 0.12 |
| Glycine (g/L) | 0.12 | 0.36 |
| Citric acid (g/L) | 0 | 0.33 |
| Amber acid (g/L) | 1.25 | 0.86 |
| Malic acid (g/L) | 0 | 1.22 |
| Lactic acid (g/L) | 3.10 | 5.26 |
| Glutamine (g/L) | 3.66 | 2.11 |
| Yeast β-glucan (mg/L) | - | 118 |

As can be seen from Table 6, through a fungus-bacterium mixed fermentation method, a fermentation broth containing high concentrations of glutamic acid and nucleotides can be obtained. Simultaneously, the fermentation broth also contains a variety of functional nutrients such as other amino acids, organic acids, β-glucan, and the like. The concentration of glutamic acid in the fermentation broth obtained at the endpoint of the fungus-bacterium fermentation can reach 80 g/L or above (up to 156 g/L), the total nucleotide concentration is up to 250 mg/L or above. The two types of substances synergistically increase umami level, enabling stronger umami than a pure glutamic acid fermentation broth, rich and harmonious taste, richer material composition, and higher functional nutritional value.

**Claims**

1. A temperature-sensitive aroma-producing *Saccharomyces cerevisiae* Y01, wherein the *Saccharomyces cerevisiae* Y01 is preserved in China Center for Type Culture Collection on May 31, 2024, with a preservation number of CCTCC NO: M 20241116.

2. Use of the temperature-sensitive aroma-producing *Saccharomyces cerevisiae* in preparation of a flavor enhancer according to claim 1, wherein the flavor enhance is prepared from a carrot juice as a main raw material through mixed fermentation of the *Saccharomyces cerevisiae* and *Corynebacterium glutamicum* CCTCC NO: M2024431.

3. A method for preparing a flavor enhancer using the temperature-sensitive aroma-producing *Saccharomyces cerevisiae* according to claim 1, comprising:

   (1) inoculating the temperature-sensitive aroma-producing *Saccharomyces cerevisiae* activated into a sterilized fermentation medium, wherein a fermentation temperature is controlled at 28±1°C, a pH range is 4.5-6.5, dissolved oxygen is maintained at 10%-30% through combined control of rotation speed and ventilation, and a fermentation time is 12 h-24 h;

(2) supplementing a carrot juice and a concentrated sugar solution which are sterilized in advance, into a fermentation tank to make total sugar reach 7% (w/v) or above, when a wet weight of the *Saccharomyces cerevisiae* reaches 50 g/L or above and the total sugar drops to 0.5% (w/v) or below, heating to 40°C-75°C, maintaining for 30 min-120 min, then rapidly cooling to 30°C, inoculating freshly activated *Corynebacterium glutamicum* CCTCC NO: M 2024431, and continuing to perform mixed fermentation for 12 h-24 h, wherein a temperature of the mixed fermentation is controlled at 30°C-33°C, dissolved oxygen is maintained at 30%-50% through combined control of rotation speed and ventilation, and pH is controlled at 6.5-8.0 during the fermentation; and

(3) heating to 100°C after the fermentation is terminated, and maintaining for 10-60 min.

4. The method according to claim 3, wherein during the mixed fermentation, when the total sugar drops to 0.2%-2.0% (w/v), fed-batch sugar supplementation is begun, and fed-batch sugar is one or a combination of two or more, in any ratio, selected from the group consisting of glucose, sucrose, fructose, corn syrup and fructose syrup, and in a sugar solution for the fed-batch sugar supplementation, in parts by weight, sugar : water = 1:1.

5. The method according to claim 3 or 4, wherein the fermentation medium uses a carrot juice as a main raw material, and comprises following components in mass ratio: 500-1000 g/kg of the carrot juice, 5.0-15 g/kg of dipotassium hydrogen phosphate, 0.05-2.0 g/kg of magnesium sulfate heptahydrate, 0.005-0.2 g/kg of ferrous sulfate heptahydrate, 0.001-0.2 g/kg of manganese sulfate monohydrate, 0-3.0 g/kg of aspartic acid, and 0-3.0 g/kg of threonine.

6. The method according to any one of claims 3 to 5, wherein in the fermentation medium, an initial total sugar content is controlled within a range of 3%-5% (w/v), an initial pH is 4.5-6.5, and the fermentation medium is sterilized after uniform mixing, under a sterilization condition of 115°C-121°C for a time of 15 min-30 min.

7. The method according to any one of claims 3 to 6, wherein a solids content of the carrot juice is 6.0°Brix.

8. A flavor enhancer prepared using the method according to claim 3, wherein the flavor enhancer comprises a product of mixed fermentation of the temperature-sensitive aroma-producing *Saccharomyces cerevisiae* and *Corynebacterium glutamicum* CCTCC NO: M 2024431 according to claim 1.

9. The flavor enhancer according to claim 8, wherein in the product, a concentration of glutamic acid is 80-156 g/L, and a concentration of total nucleotides is 250 mg/L or above.

10. The flavor enhancer according to claim 8 or 9, wherein the product further comprises β-glucan and organic acids.

FIG. 1

FIG. 2

Europäisches
Patentamt
European
Patent Office
Office européen
des brevets

# EUROPEAN SEARCH REPORT

**Application Number**

EP 25 19 1122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/248035 A1 (CHIN HSI-WEN [US] ET AL) 10 August 2023 (2023-08-10) * claims 1, 6, 10 * ----- | 1-10 | INV. A23L27/24 C12N1/18 C12R1/28 C12R1/865 |
| A | CN 110 205 255 A (UNIV JIANGNAN) 6 September 2019 (2019-09-06) * the whole document * ----- | 1-10 | |
| A | CN 116 083 259 A (SHANGHAI SIPENG TECH CO LTD) 9 May 2023 (2023-05-09) * the whole document * ----- | 1-10 | |
| A | CN 115 478 023 A (BEIJING ENHALOR INT TECH CO LTD) 16 December 2022 (2022-12-16) * the whole document * ----- | 1-10 | |

TECHNICAL FIELDS
SEARCHED      (IPC)

A23L
C12N
C12R

The present search report has been drawn up for all claims

| Place of search | Date of completion of the search | Examiner |
|---|---|---|
| The Hague | 11 November 2025 | Picout, David |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 19 1122

This annex lists the patent family members relating to the patent documents cited in  the above-mentioned European search report.
The members are as contained in the European Patent Office EDP file on
The European Patent Office is in no way liable for these particulars which are merely given for the purpose of information.

11-11-2025

| Patent document cited in search report | | Publication date | Patent family member(s) | | Publication date |
|---|---|---|---|---|---|
| US 2023248035 | A1 | 10-08-2023 | BR | 112023000702 A2 | 21-03-2023 |
| | | | CN | 116113333 A | 12-05-2023 |
| | | | EP | 4181689 A1 | 24-05-2023 |
| | | | US | 2023248035 A1 | 10-08-2023 |
| | | | WO | 2022016141 A1 | 20-01-2022 |
| CN 110205255 | A | 06-09-2019 | CN | 110205255 A | 06-09-2019 |
| | | | US | 2021321654 A1 | 21-10-2021 |
| | | | WO | 2020248962 A1 | 17-12-2020 |
| CN 116083259 | A | 09-05-2023 | NONE | | |
| CN 115478023 | A | 16-12-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202180054828
- WO 2015020292 A
- CH 201410790020
- CN 201610538817